# EUROPÄISCHE PATENTSCHRIFT

(11) **EP 0 892 681 B1**
(45) Veröffentlichungstag und Bekanntmachung des Hinweises auf die Patenterteilung: **27.02.2002**
(21) Anmeldenummer: 97920717.2
(22) Anmeldetag: 18.04.1997
(51) Int. Cl.: B05B 7/24, A61J 1/00

(54) **ZWEIKAMMER-KARTUSCHE FÜR TREIBGASFREIE DOSIERAEROSOLE**
TWO-CHAMBER CARTRIDGE FOR PROPELLANT-FREE METERED AEROSOLS
CARTOUCHE A DEUX CHAMBRES POUR DISTRIBUER SANS GAZ PROPULSEUR DES AEROSOLS DOSES

(30) Priorität: 19.04.1996 DE 19615422
(43) Veröffentlichungstag der Anmeldung: 27.01.1999
(73) Patentinhaber: Boehringer Ingelheim Pharma KG, 55218 Ingelheim am Rhein (DE)
(72) Erfinder: HOCHRAINER, Dieter, D-55411 Bingen am Rhein (DE)
(74) Vertreter: Laudien, Dieter, Dr.
(86) Internationale Anmeldenummer: EP9701958
(87) Internationale Veröffentlichungsnummer: WO9739831

(56) Entgegenhaltungen:
- EP-A- 0 577 200
- WO-A-90/07319
- WO-A-96/03344
- FR-A- 1 112 540
- US-A- 3 874 380

## Beschreibung

Die vorliegende Erfindung betrifft eine Zweikammer-Kartusche für Flüssigkeiten, insbesondere für Arzneimittelformulierungen zur Anwendung in treibgasfreien Dosieraerosolen.

In der internationalen Patentanmeldung WO91/14468 "Atomizing Device and Methods" ist eine Vorrichtung zur treibgasfreien Verabreichung einer dosierten Menge eines flüssigen Arzneimittels zur inhalativen Anwendung beschrieben. Eine weiterentwickelte Ausführungsform ist beispielsweise in der WO 97/12687 beschrieben. Für solche Anwendungszwecke ist es erforderlich, die wirkstoffhaltigen Lösungen so in Behälter abzufüllen, daß nur geringe Luft- und Gasreste mit eingeschlossen werden. Gasblasen würden zu Unsicherheiten bei der exakten Dosierung des Wirkstoffes führen. Solche Behälter sind beispielsweise in der internationalen Patentanmeldung WO 96/06011 offenbart. ***Die dort beschriebenen Behälter werden durch eine Verschlußkappe verschlossen. Die Verschlußkappe weist eine Einrichtung auf, die einen Teil der Flüssigkeit aus dem Inneren des Behälters während des Verschließens verdrängt. Diese*** Behälter sind vor allem für solche Arzneimittel geeignet, die in Form einer wässerigen oder ethanolischen Lösung über einen längeren Zeitraum lagerstabil sind.

***Die WO 96/03344 beschreibt Handzerstäuber bestehend aus einem handbetätigbaren Sprühkopf und einer Vorratsflasche zum Versprühen von mit Haushaltschemikalien beinhaltenden Lösungen. In den Zerstäuber kann ein austauschbarer Einsatz eingesetzt werden, in dem sich die entsprechende Chemikalie befindet. Beim Aufschrauben des Sprühkopfes auf die Vorratsflasche wird der Einsatz geöffnet und das Chemikalienkonzentrat ergießt sich in die Vorratsflasche.***

***Die US 3 874 380 beschreibt eine Nasensprayvorrichtung, die eine Vorratsflasche für lyophilisierte biologisch aktive Substanzen in Form einer Tablette enthält. Durch Einspritzen einer Flüssigkeit mit einer Spritze wird die Wirkstofflösung hergestellt, die anschließend in die gleiche Spritze aufgezogen wird und von dort aus über eine Düse verstäubt wird.***

Für Wirkstoffe, die sich bereits nach wenigen Monaten in ihren Lösungen zersetzen, gab es bislang keine geeigneten Behälter, die eine kommerzielle Anwendung solcher empfindlicher Zubereitungen in treibgasfreien Dosieraerosolen ermöglicht hätten.

Die Erfindung betrifft nunmehr eine Kartusche, die zwei Kammern zur getrennten Aufbewahrung von Wirkstoff und Lösungsmittel aufweist. Die Kartusche ist derart ausgestaltet, daß beim Einsetzen der Kartusche in eine Vorrichtung zur Erzeugung des Aerosols die Kammer, die den Wirkstoff enthält, mit einer Kanüle durchstochen wird, wobei der Wirkstoff mit dem Lösemittel in Kontakt kommt und aufgelöst wird. Durch die getrennte Aufbewahrung von Wirkstoff und Lösemittel kann die Lagerzeit der Arzneimittelzubereitung wesentlich verlängert werden. Der Wirkstoff kann als Pulver, Granulat oder auch in Form einer Tablette in der Kammer vorhanden sein. Ebenso können pharmakologisch verträgliche Hilfsstoffe zugegen sein. Bevorzugt werden allgemein solche galenischen Formulierungen, die ein einfaches Auflösen des Wirkstoffs in dem Lösemittel fördern. Bei Tabletten können Hilfstoffe zugesetzt sein, die ein besseres Auflösen der Tablette bewirken. Es können ebenso Hilfsstoffe zugesetzt sein, die die Stabilität der Wirkstoffe erhöhen. In manchen Fällen kann der Wirkstoff auch in gelöster Form in der Kammer vorhanden sein, sofern der Wirkstoff in dem Lösemittel stabil ist und das Lösemittel mit dem Lösemittel in der anderen Kammer, im weiteren auch Behälter genannt, mischbar ist.

Nachfolgend wird die Erfindung anhand konkreter Ausführungsbeispiele näher erläutert.

Figur 1 zeigt einen axialen Schnitt entlang der Längsachse der erfindungsgemäßen Kartusche (1) mit der Kammer (2) zur Aufnahme des Wirkstoffs, wobei die Kammer (2) integraler Bestandteil der Verschlußkappe (3) ist.

Figur 2 zeigt eine weitere Ausführungsform der Verschlußkappe (3) mit Kammer (2) in geschlossenem Zustand der Kartusche, wobei der Behälter (4) nur angedeutet ist.

Die Figuren 3a bis 3c zeigen weitere Ausführungsformen der erfindungsgemäßen Verschlußkappe (3) mit Kammer (2).

Figur 4 zeigt einen Schnitt entlang der Längsachse einer Ausführungsform der erfindungsgemäßen Verschlußkappe, worin die Kammer (2) eine Minitablette (16a) als Wirkstoffvorrat enthält.

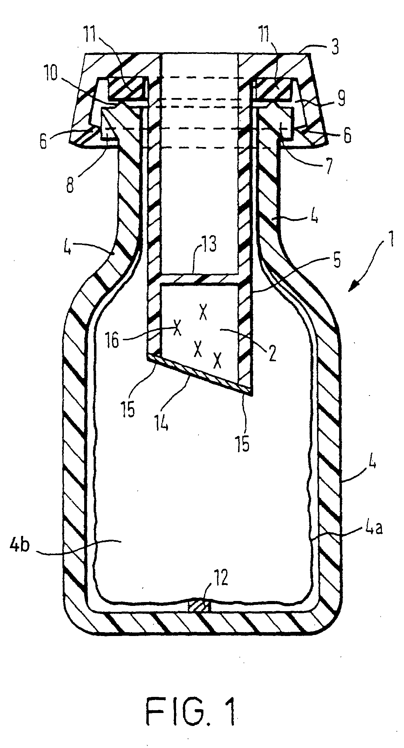

In Figur 1 ist die erfindungsgemäße Kartusche (1) bestehend aus einem Behälter (4) und einer Verschlußkappe (3) dargestellt. Die Verschlußkappe weist eine Einrichtung - hier in Form eines eintauchenden Stutzens (5) - auf, durch die während des Verschließvorganges ein Teil des Inhalts aus dem Behälter (4) verdrängt und der Behälter praktisch luftblasenfrei gefüllt wird. Ein innenliegender umlaufender Wulst (6) am unteren Rand der Verschlußkappe (3) rastet in der Verschlußposition unterhalb eines an der Außenseite des Behälterhalses umlaufenden zylindrischen Ringes (7) ein. In der Verschlußposition wird der Zwischenraum zwischen dem flachen Teil der Verschlußkappe (3) und dem oberen Rand des Behälterhalses, der ggf. zur besseren Abdichtung mit einer umlaufenden Rippe (10) versehen ist, durch einen Dichtungsring (11) ausgefüllt und damit der Innenraum des Behälters (3) abgedichtet. Der Innendurchmesser des Dichtungsrings (11) wird zweckmäßig so gewählt, daß er an dem Stutzen (5) eng anliegt. Die Entlüftungsöffnung(en) (8) kann (können) sich auch an anderen Stellen der Kappenaußenseite befinden, etwa seitlich in dem zylindrischen Teil der Kappe.

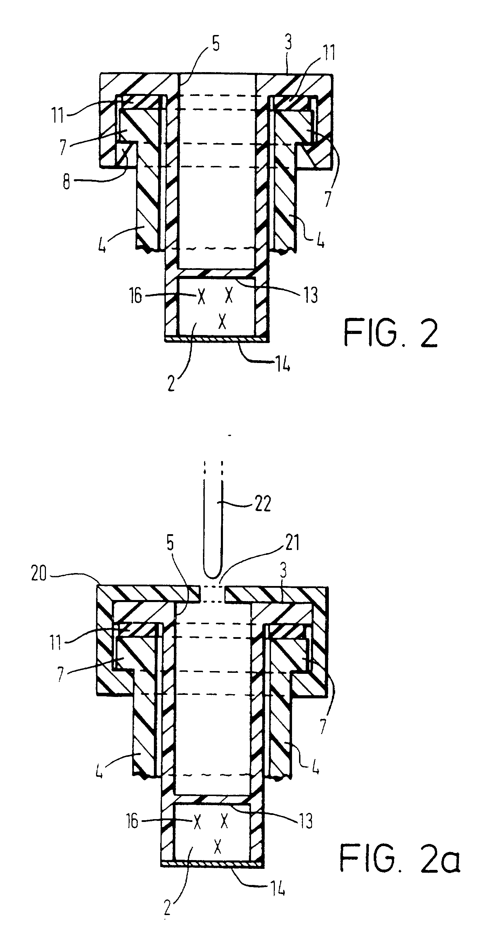

In einer anderen Ausführungsform Fig. 2a wird die Verschlußkappe (3) durch eine Hülse (20) aus Aluminium, die gekrimpt wird, verschlossen. Die Hülse (20) ist derart ausgestaltet, daß sie eine zentrale Öffnung (21) zur Durchführung der Kanüle (22) aufweist.
Diese Öffnung kann durch ein Septum zum Schutz vor Staub und anderen Verunreinigungen verschlossen sein. Diese Verschlußtechnik ist beispielsweise bei Injektionsampullen bekannt.

In einer besonderen Ausführungsform enthält der Behälter (4) einen kollabierbaren Innenbehälter (4a) aus einem flexiblen Material. Der Innenbehälter kann in einer bevorzugten Ausführungsform am unteren Teil des Behälters (4) durch eine Vorrichtung (12) befestigt sein.

Die Kammer (2) befindet sich im unteren Teil des Stutzens (5) wobei die Kammer durch eine Abtrennung, beispielsweise in Form eines Septums (13), nach außen und durch eine Abtrennung, beispielsweise in Form einer Folie (14), zum Behälterinnenraum (4b) abgeschlossen ist. Das Septum (13) und die Folie (14) sind aus einem Material gefertigt, welches durch eine Kanüle mit spitzer oder abgerundeter Spitze leicht durchstoßen werden kann. Das Septum (13) ist bevorzugt aus einem Material gefertigt, das auch bei durchgestoßener Kanüle den Innenraum (4b) nach außen abdichtet. Üblicherweise bestehen die Abtrennungen aus dünnen Kunststoff- oder Aluminiumfolien. In einer Ausführungsform kann das Septum (13) an seiner Verbindung zur Seitenwand des Stutzens (5) Schwachstellen aufweisen, so daß beim Durchstoßen der Abtrennung dieses an den Schwachstellen aufreißt. Bevorzugt ist die Folie (14) als angeschweißte diffusionsdichte Siegelfolie ausgeführt, die beim Durchstoßen abreißt und der Wirkstoff in den Innenraum (4b) des Behälters gelangt. Die Schwachstellen können auch im Bereich der unteren Seitenwand des Stutzens (5) angeordnet sein, so daß der untere Teil der Seitenwand des Stutzens mit abreißt.

Die Position der Abtrennung (des Septums (13)) kann in weiten Bereichen des Innenraums des Stutzens (5) variieren, bevorzugt ist sie jedoch in Abhängigkeit der Menge an Wirkstoff (16) so angeordnet, daß der durch beiden Abtrennungen (13) und (14) gebildete Innenraum, außer dem Pulver, eine möglichst geringe Menge an Gasvolumen (Luft) umfaßt.

Figur 2 zeigt ebenfalls einen axialen Schnitt eines Behälterhalses mit aufgesetzten Verschlußkappe (3), wobei die Kammer (2) anders gestaltet ist.

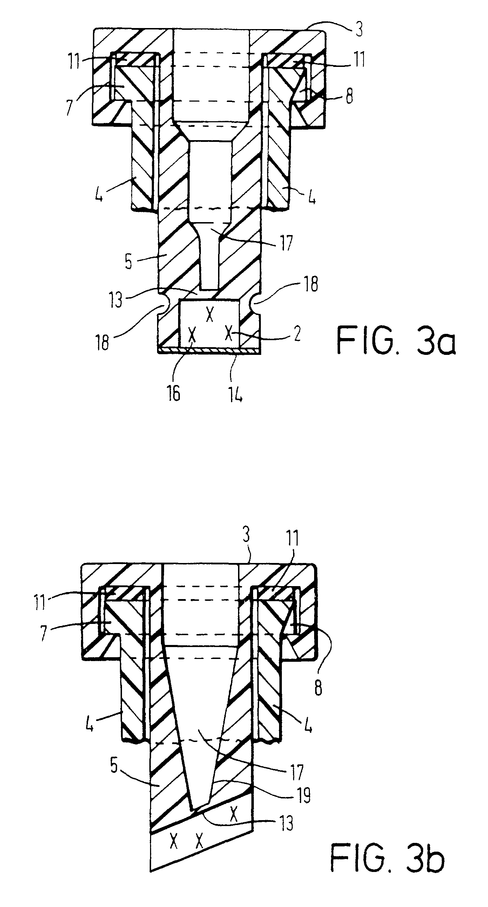

Die Figur 3a zeigt eine weitere Ausführungsform der erfindungsgemäßen Verschlußkappe, bei der der Innenraum des Eintauchstutzens derart gestaltet ist, daß eine Führung (17) für eine Kanüle zur Flüssigkeitsentnahme ausgebildet ist. Im vorliegenden Fall sind die Entlüftungsöffnungen (8) am oberen Teil des Behälters (4) angebracht. Wie bereits beschrieben, können die Entlüftungsöffnungen alternativ auch an der Verschlußkappe angeordnet sein. Die Kammer (2) zur Aufnahme des Wirkstoffs ist separat im unteren Teil des Stutzens (5) angeordnet. Anstelle einer durchstechbaren Abtrennung (14) können Schwachstellen (18) vorhanden sein, so daß die Kammer beim Durchstoßen der Abtrennung (13) durch Druck auf die Abtrennung (14) an den Schwachstellen (18) abreißt. Bei dieser Ausführungsform kann die Abtrennung (14) als Boden des Stutzens (5) ausgeformt sein.

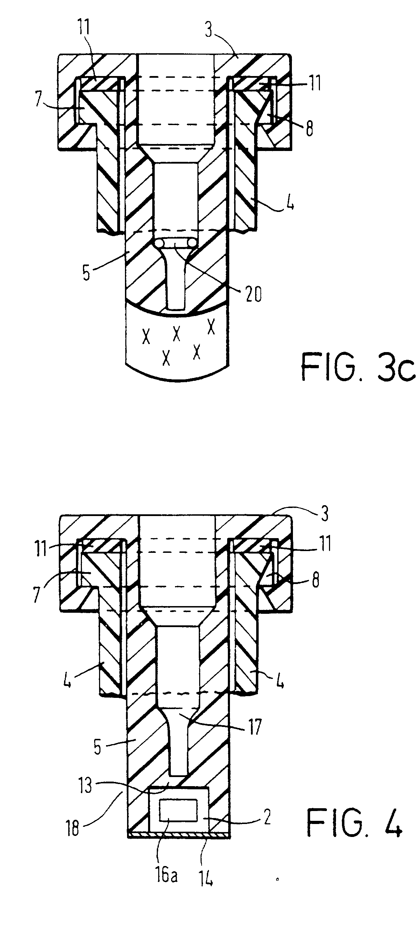

Die Figuren 3b, 3c zeigen andere Ausführungsformen hinsichtlich der Ausgestaltung des eintauchenden Stutzens (5) sowie der Führung (17) für die Kanüle zur Entnahme der Flüssigkeit.

Die Figur 3b zeigt eine Ausführungsform bei der die Führung (17) in eine Presspassung (19) übergeht. Der Preßsitz ist hinsichtlich Durchmesser und Länge so ausgelegt, daß einerseits der Widerstand zum Durchschieben der Kanüle gering gehalten ist und andererseits eine ausreichende Dichtwirkung zwischen Stutzen und Kanüle erreicht wird.

Fig. 3c zeigt eine Ausführungsform mit einer elastischen O-Ring-Dichtung (20) zwischen Stutzen und einstechender Kanüle, wobei die Kanüle nicht abgebildet ist.
Nicht dargestellt ist die Vorrichtung, die ein unbeabsichtigtes Lösen des O-Ringes verhindert.

Wie in Fig. 3b und 3c gezeigt, kann das untere Ende des Eintauchstutzens mit der Abtrennung (14) zweckmäßigerweise abgeschrägt sein, vorzugsweise um 20° bis 60° gegenüber der Stutzenachse. Dadurch wird ein Durchstoßen der Abtrennung mit einer "stumpfen" Kanüle, deren Stirnfläche senkrecht zur Kanülenachse steht, erleichtert. Vorteile einer "stumpfen" gegenüber einer "spitz angeschliffenen" Kanüle liegen in der geringen Verletzungsgefahr für den Anwender, in dem geringeren Bearbeitungsaufwand zur Herstellung der Kanülen-Stirnfläche und der beim Einführen der Kanüle geringeren Gefahr des Partikelabriebs an der Stutzenwandung.

Wie in Figur 4 dargestellt, die weitestgehend der Figur 3a entspricht, enthält die Kammer (2) den Wirkstoff in Form einer kleinen Tablette. Gegenüber einem pulverförmigen Wirkstoff kann der Wirkstoff in Form der erfindungsgemäßen Minitablette wesentlich einfacher in die Kammer (2) eingebracht werden, ebenso bietet eine Tablette Vorteile wenn das Septum (13) mit einer Kanüle durchstochen wird und anschließend die Tablette (16a) durch die Folie (14) gestoßen wird. Zum einen ist gewährleistet, daß die relativ harte Tablette die Kanüle nicht verstopft, zum anderen ist sichergestellt, daß die gesamte Wirkstoffmenge der Kammer in den Behälter (4) gelangt. Bei den heute üblicherweise in Dosieraerosolen eingesetzten hochwirksamen Arzneimitteln ist eine genau eingestellte Wirkstofflösung im Sinne der Arzneimittelsicherheit unbedingt erforderlich. Ferner wird beim Befüllen der Kammer (2) mit einer Tablette die Siegelfläche nicht mit Staub kontaminiert, wie dies bei Befüllen mit Pulver der Fall wäre.

Die erfindungsgemäße Tablette weist einen Durchmesser zwischen 2 und 3 mm, bevorzugt zwischen 2,2 und 2,3 mm sowie eine Länge zwischen 1,8 und 3,5 mm auf. Die erfindungsgemäße Tablette weist eine Druckfestigkeit zwischen 2 und 10 N/mm² auf. Die Messung der Druckfestigkeit erfolgt so, daß die Tabletten zwischen ebenen Flächen eingespannt werden und die Kraft bis zum Zerbrechen der Tabletten erhöht wird. Die Tabletten waren so eingespannt, daß sie längs zweier Mantellinien die ebenen Flächen berühren (nicht mit Boden und Deckfläche). Als Druckfestigkeit gibt man die Kraft geteilt durch die Querschnittsfläche (Durchmesser mal Länge der zylindrischen Tablette) an.

Die erfindungsgemäßen Tabletten bestehen aus dem Wirkstoff und üblichen Tablettierhilfsstoffen. Bevorzugte Wirkstoffe sind solche, die in niedriger Dosierung angewendet werden können, beispielsweise bis zu 100 Mikrogramm Wirkstoff pro Einzelanwendung. Genannt werden beispielhaft Atrovent, Anticholinergica, β-Sympatikomimetika, z. B. Formoterol. Bevorzugte Hilfsstoffe sind Lactose (200 mesh), Glukose (200 mesh) und Formtrennmittel.

Der erfindungsgemäße Behälter weist ein Lösungsmittelvolumen von 4 ml auf, so daß mit einer Minitablette, die 20 mg wiegt, 0,5 %ige Wirkstofflösungen hergestellt werden können. Als Lösemittel werden bevorzugt Wasser oder Ethanol oder deren Gemische verwendet. Geeignet sind ferner andere physiologisch verträgliche Lösemittel.

Für die Entnahme von Flüssigkeit aus der erfindungsgemäßen Kartusche (1) werden die Abtrennungen (13 und 14) mit einer Kanüle durchstochen. Bevorzugt sind Ausführungsformen, bei denen der Behälter (4) einen leicht verformbaren Innenbeutel (4a) aufweist und das Ende der Kanüle sich in halber Höhe des Behälters befindet, wenn die Flüssigkeit entnommen wird. In diesem Fall wirken sich Luftblasen am wenigsten störend aus. Bevorzugt wird als Wirkstoffdepot die erfindungsgemäße Minitablette (16a) verwendet.

Behälter und Verschlußkappe werden im allgemeinen aus Kunststoff gefertigt. Da die eingefüllte Flüssigkeit praktisch nicht kompressibel ist, muß das System aus Behälter und Verschlußkappe bei der Ausdehnung der Flüssigkeit in der Wärme ausreichend verformbar sein. Ebenso müssen bei der Entnahme der Flüssigkeit die Wandungen des Behälters ausreichend nachgeben bzw. zusammenfallen. Die Abtrennung (13) besteht im allgemeinen aus einer dünnen Kunstofffolie. Bevorzugt ist die Abtrennung (14) aus einer dünnen und beschichteten Aluminium gefertigt, die aufgesiegelt wird.

Für die Herstellung solcher Behälter wie auch für die Verschlußkappe stehen dem Fachmann geeignete Kunststoffe - beispielsweise aus Polyethylen oder bevorzugt Polypropylen - zur Verfügung.

Die erfindungsgemäße Kartusche für Arzneimittelformulierungen für ein Inhalationsgerät soll eine lange Lagerfähigkeit aufweisen. Hierzu ist es erforderlich, daß das Lösungsmittel aus dem Behälterinnenraum (4a) vor Gebrauch nicht in die Kammer (2), die den Wirkstoff enthält, diffundieren kann. Neben einer ausreichenden Wandstärke der Kammer, kann zusätzlich eine Aluminiumbeschichtung auf die Außenflächen oder die Innenflächen der Kammer (2) aufgebracht sein. Es ist hervorzuheben, daß das Einsetzen der Kartusche mit der Kammer (2) in den Inhalator für den Patienten keinen weiteren Handgriff erfordert als das Einsetzen einer gewöhnlichen Kartusche.

## Patentansprüche

1. Kartusche für treibgasfreie Dosieraerosole bestehend aus einem Behälter (4) mit Verschlußkappe (3) für die Abfüllung von Flüssigkeiten, einem am unteren Rand der Verschlußkappe (3) ausgebildetem innenliegenden umlaufenden Wulst (6), der in Verschlußposition unterhalb eines an der Außenseite des Behälters (4) umlaufenden Rings (7) einrastet, und einem mit der Verschlußkappe (3) verbundenem Stutzen (5), der während des Aufschiebens der Verschlußkappe (3) auf den Hals des Behälters (4) einen Teil des Behälterinhalts verdrängt, **dadurch gekennzeichnet, daß** der Stutzen (5) mindestens eine Kammer (2) enthält, die durch zwei durchstechbare Abtrennungen (13) und (14) gegenüber der Außenumgebung und dem Innenraum des Behälters (4) abgeschlossen ist.

2. Kartusche nach Anspruch 1, **dadurch gekennzeichnet, daß** die Kammer (2) ein Arzneimittel enthält.

3. Kartusche nach Anspruch 2, **dadurch gekennzeichnet, daß** der Behälter (4) ein Lösemittel enthält.

4. Kartusche nach Anspruch 1 bis 3, **dadurch gekennzeichnet, daß** die Kammer (2) einen für die inhalative Applikation geeigneten Wirkstoff enthält.

5. Kartusche nach Anspruch 1 bis 4, **dadurch gekennzeichnet, daß** der Wirkstoff in Form einer Tablette vorliegt.

6. Kartusche nach einem der vorhergehenden Ansprüche, **dadurch gekennzeichnet, daß** die Abtrennung der Kammer (2) nach Außen als ein Septum (13) und zum Innenbehälter als eine Siegelfolie (14) ausgebildet ist.

7. Kartusche nach einem der Ansprüche 1 bis 6, **dadurch gekennzeichnet, daß** die Kammer (2) an ihren Verbindungsstellen zum Stutzen (5) Schwachstellen aufweist, so daß beim Durchstoßen des Septums (13) durch Druck auf die Kammer (2) diese an den Schwachstellen abreißt.

8. Verschlußkappe (3) für Behälter (4) für treibgasfreie Dosieraerosole, mit einem am unteren Rand der Verschlußkappe (3) ausgebildetem innenliegenden umlaufenden Wulst (6), der in Verschlußposition unterhalb eines an der Außenseite des Behälters (4) umlaufenden Rings (7) einrastet, und einem mit der Verschlußkappe (3) verbundenem Stutzen (5), der während des Aufschiebens der Verschlußkappe (3) auf den Hals des Behälters (4) einen Teil des Behälterinhalts verdrängt, **dadurch gekennzeichnet, daß** der Stutzen (5) mindestens eine Kammer (2) enthält, die durch zwei durchstechbare Abtrennungen (13) und (14) gegenüber der Außenumgebung und dem Innenraum des Behälters (4) abgeschlossen ist.

9. Verschlußkappe nach Anspruch 8, **dadurch gekennzeichnet, daß** die Kammer (2) einen für die inhalative Applikation geeigneten Wirkstoff enthält.

10. Verschlußkappe nach Anspruch 8 oder 9, **dadurch gekennzeichnet, daß** der Wirkstoff in Form einer Tablette vorliegt.

11. Verschlußkappe nach einem der vorhergehenden Ansprüche 8 bis 10, **dadurch gekennzeichnet, daß** die Abtrennung der Kammer (2) nach Außen als ein Septum (13) und zum Innenbehälter als eine Siegelfolie (14) ausgebildet ist.

12. Verschlußkappe nach einem der Ansprüche 8 bis 10, **dadurch gekennzeichnet, daß** die Kammer (2) an ihren Verbindungsstellen zum Stutzen (5) Schwachstellen aufweist, so daß beim Durchstoßen des Septums (13) durch Druck auf die Kammer (2) diese an den Schwachstellen abreißt.

13. Verschlußkappe nach einem der vorherigen Ansprüche 8 bis 12, **dadurch gekennzeichnet, daß** das Septum aus einem elastischem Material gefertigt ist, so daß der Behälterinnenraum auch nach dem Durchstoßen der Kanüle nach außen abgedichtet ist.

14. Minitablette als Wirkstoffträger für treibgasfreie Dosieraerosole zum einbringen in eine Kammer (2) gemäß einem der Ansprüche 1 oder 8, enthaltend einen Wirkstoff für die inhalative Applikation sowie gegebenenfalls pharmakologisch unbedenkliche Hilfsstoffe, **dadurch gekennzeichnet, daß** sie eine Härte zwischen 2 und 10 N/mm² aufweist.

15. Minitablette nach Anspruch 14, **dadurch gekennzeichnet, daß** sie einen Durchmesser zwischen 2 und 3 mm und eine Länge zwischen 1,0 und 4,0 mm aufweist.

16. Minitablette nach einem der Ansprüche 14 bis 15 zur Herstellung einer Wirkstofflösung.

## Claims

1. Cartridge for propellant-free metering aerosols consisting of a container (4) with closure cap (3) for holding liquids, an internal encircling bead (6) formed on the lower edge of the closure cap (3) which in the closed position engages below a ring (7) running around the outside of the container (4), and a connector (5) connected to the closure cap (3), which displaces some of the contents of the container as the closure cap (3) is pushed onto the neck of the container (4), **characterised in that** the connector (5) contains at least one chamber which is sealed off from the outside and from the interior of the container (4) by two pierceable partitions (13) and (14).

2. Cartridge according to claim 1, **characterised in that** the chamber (2) contains a pharmaceutical composition.

3. Cartridge according to claim 2, **characterised in that** the container (4) contains a solvent.

4. Cartridge according to claims 1 to 3, **characterised in that** the chamber (2) contains an active substance which is suitable for administration by inhalation.

5. Cartridge according to claims 1 to 4, **characterised in that** the active substance is present in the form of a tablet.

6. Cartridge according to one of the preceding claims, **characterised in that** the means separating the chamber (2) from the outside are in the form of a septum (13) and the means separating it from the inner container are in the form of a sealing film (14).

7. Cartridge according to one of claims 1 to 6, **characterised in that** the chamber (2) has frangible points at its points of connection to the connector (5), so that when the septum (13) is pierced by pressure on the chamber (2) the latter tears at the frangible points.

8. Closure cap (3) for containers (4) for propellant-free metering aerosols, having an internal encircling bead (6) formed on the lower edge of the closure cap (3) which in the closed position engages below a ring (7) running around the outside of the container (4), and a connector (5) connected to the closure cap (3), which displaces some of the contents of the container as the closure cap (3) is pushed onto the neck of the container (4), **characterised in that** the connector (5) contains at least one chamber which is sealed off from the outside and from the interior of the container (4) by two pierceable partitions (13) and (14).

9. Closure cap according to claim 8, **characterised in that** the chamber (2) contains an active substance suitable for administration by inhalation.

10. Closure cap according to claim 8 or 9, **characterised in that** the active substance is present in the form of a tablet.

11. Closure cap according to one of the preceding claims 8 to 10, **characterised in that** the partition separating the chamber (2) from the outside is in the form of a septum (13) and the partition from the internal container is constructed as a sealing film (14).

12. Closure cap according to one of claims 8 to 10, **characterised in that** the chamber (2) has frangible points at its points of connection to the connector (5), so that when the septum (13) is pierced by pressure on the chamber (2) the latter tears at the frangible points.

13. Closure cap according to one of the preceding claims 8 to 12, **characterised in that** the septum is made from a resilient material so that the interior of the container is sealed off from the outside even after being pierced by the cannula.

14. Minitablet as an active substance carrier for propellant-free metering aerosols for placing in a chamber (2) according to one of claims 1 or 8, containing an active substance for administration by inhalation and optionally pharmacologically acceptable excipients, **characterised in that** it has a hardness of between 2 and 10 N/mm².

15. Minitablet according to claim 14, **characterised in that** it is between 2 and 3 mm in diameter and between 1.0 and 4.0 mm long.

16. Minitablet according to one of claims 14 and 15 for preparing a solution of active substance.

## Revendications

1. Cartouche pour aérosols dosés sans gaz propulseur consistant en un récipient (4) avec un capuchon de fermeture (3) pour l'introduction de liquides, un renflement (6) périphérique situé à l'intérieur, formé au niveau du bord inférieur du capuchon de fermeture (3), qui, en position de fermeture, s'enclenche sous un anneau (7) périphérique sur le côté externe du récipient (4), et une tubulure (5) reliée au capuchon de fermeture (3) qui, pendant la mise en place du capuchon de fermeture (3) sur le col du récipient (4), déplace une partie du contenu du récipient, **caractérisée en ce que** la tubulure (5) contient au moins une chambre (2) qui est isolée par deux séparations perforables (13) et (14) de l'environnement extérieur et de la cavité interne du récipient (4).

2. Cartouche selon la revendication 1 **caractérisée en ce que** la chambre (2) contient un médicament.

3. Cartouche selon la revendication 2 **caractérisée en ce que** le récipient (4) contient un solvant.

4. Cartouche selon les revendications 1 à 3 **caractérisée en ce que** la chambre (2) contient un principe actif approprié à l'application par inhalation.

5. Cartouche selon les revendications 1 à 4 **caractérisée en ce que** le principe actif est sous forme d'un comprimé.

6. Cartouche selon l'une des revendications précédentes **caractérisée en ce que** la séparation de la chambre (2) d'avec l'extérieur est sous forme d'un septum (13) et d'avec le récipient interne sous forme d'une feuille de scellement (14).

7. Cartouche selon l'une des revendications 1 à 6 **caractérisée en ce que** la chambre (2) comporte des points faibles au niveau de ses sites de liaison avec la tubulure (5) de sorte que, lors de la perforation du septum (13) par pression sur la chambre (2), celle-ci se rompt au niveau des points faibles.

8. Capuchon de fermeture (3) pour récipient (4) pour aérosols dosés sans gaz propulseur avec un renflement (6) périphérique situé à l'intérieur, formé au niveau du bord inférieur du capuchon de fermeture (3), qui, en position de fermeture, s'enclenche sous un anneau (7) périphérique sur le côté externe du récipient (4), et une tubulure (5) reliée au capuchon de fermeture (3) qui, pendant la mise en place du capuchon de fermeture (3) sur le col du récipient (4), déplace une partie du contenu du récipient, **caractérisé en ce que** la tubulure (5) contient au moins une chambre (2) qui est isolée par deux séparations perforables (13) et (14) de l'environnement extérieur et de la cavité interne du récipient (4).

9. Capuchon de fermeture selon la revendication 8 **caractérisé en ce que** la chambre (2) contient un principe actif approprié à l'application par inhalation.

10. Capuchon de fermeture selon la revendication 8 ou 9 **caractérisé en ce que** le principe actif est sous forme d'un comprimé.

11. Capuchon de fermeture selon l'une des revendications précédentes 8 à 10 **caractérisé en ce que** la séparation de la chambre (2) d'avec l'extérieur est sous forme d'un septum (13) et d'avec le récipient interne sous forme d'une feuille de scellement (14).

12. Capuchon de fermeture selon l'une des revendications 8 à 10 **caractérisé en ce que** la chambre (2) comporte des points faibles au niveau de ses sites de liaison avec la tubulure (5) de sorte que, lors de la perforation du septum (13) par pression sur la chambre (2), celle-ci se rompt au niveau des points faibles.

13. Capuchon de fermeture selon l'une des revendications précédentes 8 à 12 **caractérisé en ce que** le septum est en un matériau élastique de sorte que la cavité interne du récipient est étanchéifiée par rapport à l'extérieur même après la perforation de la canule.

14. Minicomprimé comme support de principe actif pour aérosols dosés sans gaz propulseur destiné à être introduit dans une chambre (2) selon l'une des revendications 1 et 8 contenant un principe actif pour l'application par inhalation et éventuellement des adjuvants pharmacologiquement acceptables, **caractérisé en ce qu'**il présente une dureté située entre 2 et 10 N/mm².

15. Minicomprimé selon la revendication 14 **caractérisé en ce qu'**il présente un diamètre situé entre 2 et 3 mm et une longueur située entre 1,0 et 4,0 mm.

16. Minicomprimé selon l'une des revendications 14 et 15 pour la préparation d'une solution de principe actif.
